# FASCICULE DE BREVET EUROPEEN

(11) **EP 3 270 882 B1**
(45) Date de publication et mention de la délivrance du brevet: **07.06.2023**
(21) Numéro de dépôt: 16713970.8
(22) Date de dépôt: 17.03.2016
(51) Int. Cl.: A61L 27/52, C08J 3/12, A61P 19/04, A61P 19/00, A61P 17/00, A61K 8/65, A61K 8/02, A61L 27/24, A61Q 19/08

(54) **SUSPENSIONS DE COLLAGENE INJECTABLES, LEUR PROCEDE DE PREPARATION ET LEURS UTILISATIONS, NOTAMMENT POUR LA FORMATION DE MATRICES DENSES DE COLLAGENE**
INJIZIERBARE KOLLAGENSUSPENSIONEN, HERSTELLUNGSVERFAHREN DAFÜR UND VERWENDUNGEN DAVON, INSBESONDERE ZUR AUSBILDUNG DICHTER KOLLAGENMATRICES
INJECTABLE COLLAGEN SUSPENSIONS, THE PREPARATION METHOD THEREOF, AND THE USES THEREOF, PARTICULARLY FOR FORMING DENSE COLLAGEN MATRICES

(30) Priorité: 17.03.2015 FR 1552200
(43) Date de publication de la demande: 24.01.2018
(73) Titulaire: Sorbonne Université, 75006 Paris (FR); Centre National de la Recherche Scientifique CNRS, 75794 Paris Cedex 16 (FR)
(72) Inventeur: NASSIF, Nadine, 75005 Paris (FR); MOREIRA MARTINS FERNANDES, Francisco Miguel, 75013 Paris (FR); BOISSIERE, Cédric, 91140 Villebon sur Yvette (FR); SANCHEZ, Clément, 91140 Bures-sur-Yvette (FR); GIRAUD-GUILLE, Marie-Madeleine, 75012 Paris (FR)
(74) Mandataire: Grosset-Fournier, Chantal Catherine
(86) Numéro de dépôt international: PCT/FR2016/050599
(87) Numéro de publication internationale: WO 2016/146954

(56) Documents cités:
- WO-A1-2015/053629
- WO-A1-2015/053629
- CN-A- 104 059 954
- JP-A- 2006 016 381
- JP-A- 2006 016 381
- JP-A- 2009 139 335
- JP-A- 2009 139 335
- US-A- 3 393 080
- US-A- 3 393 080
- US-A- 5 667 961
- US-A- 5 667 961
- US-A- 5 739 102
- US-A- 5 739 102
- US-A1- 2004 137 051
- US-A1- 2013 190 479
- US-A1- 2013 190 479
- TANIZAKI YUTA ET AL: "Cellular characterization of thrombocytes inXenopus laeviswith specific monoclonal antibodies", EXPERIMENTAL HEMATOLOGY, vol. 43, no. 2, 1 février 2015 (2015-02-01), pages 125-136, XP029137245, ISSN: 0301-472X, DOI: 10.1016/J.EXPHEM.2014.10.005

## Description

La présente demande décrit la préparation de suspensions de collagène injectables, leur procédé de préparation et leurs utilisations, notamment pour la formation de matrices denses de collagène.

La part des collagènes dans le corps humain est prépondérante, les collagènes fibrillaires participent à la structuration des matrices extracellulaires. On trouve le collagène de type I en abondance dans les tissus de soutien, les vaisseaux, le derme, les tendons et ligaments, la cornée et l'os. Il reste donc un candidat de choix, comme polymère naturel constitutif de substituts tissulaires, par ses propriétés hémostatiques, son faible pouvoir immunogène et ses implications dans le comportement cellulaire. Ainsi, dans le contexte actuel, différents types de substituts à base de collagène sont déjà proposés au niveau d'applications industrielles. De nombreuses méthodes ont été proposées dans la littérature pour la formulation de collagène (Friess et al., Eur. J. Pharma. Biopharma. 1998, 45, 113-136), mais ces matrices sont généralement peu concentrées en collagène.

La mise en oeuvre de particules de collagène à partir de collagène acido-soluble comme moyen permettant l'obtention de matériaux collagèniques concentrés a été proposée par plusieurs méthodes. Morson (GB2274458) a démontré que le collagène acido-soluble peut être séché par voie aérosol pour obtenir des particules utilisables dans la clarification de boissons fermentées. Néanmoins, les conditions opératoires préconisées dans cette demande, notamment des températures de séchage de 120°C, impliquent la dénaturation des triples hélices de collagène.

D'autres travaux (EP2599820) proposent des méthodes de précipitation du collagène à partir de l'utilisation des solvants organiques hydrophiles tels que l'éthanol, l'acétone et l'éther diéthylique. Par cette méthode, les auteurs parviennent à précipiter des particules de collagène de taille micronique. Néanmoins, l'utilisation des solvants organiques, et plus particulièrement la présence des traces de ces composés dans les matériaux obtenus peuvent poser des problèmes de toxicité lors de l'injection des formulations.

Le document US3393080 décrit un procédé de préparation d'une dispersion stable d'une nouvelle forme colloïdale microcristalline submicronique de tropocollagène comprenant de 25 à 35% de solvant organique tel que le diméthylsulfoxyde ou l'isopropanol. Les compositions de collagène, du fait de la présence d'une quantité importante de solvant organique, résistent aux contaminations bactériennes. Un autre avantage des compositions de collagène de ce document est la possibilité d'obtenir des fibres pouvant être filées à sec après élimination des solvants organiques. Les exemples de ce document décrivent des dispersions de collagène dans l'isopropanol utiles dans les pommades et médicaments topiques anti-aseptiques, des produits de collagène lyophilisé utiles pour la formation de pansements chirurgicaux ou de serviettes hygiéniques ou de produits associés à de la cellulose.

Des formulations sous forme de billes basées sur des techniques d'émulsion dans un mélange eau/huile (Hsu et al., Biomaterials, 1999, 20, 1931-1936) ou eau/solvants organiques (US4565580) dans des conditions de non dénaturation de la protéine ont aussi été proposées. Cependant, des traces d'huile ou de solvants sont associées aux billes formées. Une alternative est la technique de séparation de phase induite thermiquement (thermally induced phase séparation (TIPS)) dont les gouttes de la solution sont introduites dans un bain d'azote liquide suivi par une étape de lyophilisation (Keshaw, et al., Acta. Biomater. 2010, 6, 1158-1166). Dans tous les cas, la fragilité des billes de collagène formées conduit à une étape de réticulation par des agents chimiques (aldéhydes, carbodiimides) dont la toxicité pose problème. En effet, dans ces travaux, les molécules de collagène de type I sont en solution, à pH acide, à faible concentration (environ 1 mg/ml).

Ainsi, un but de la présente demande est de décrire des suspensions de collagène formant *in vivo* des matrices denses de collagène.

Un autre but de la présente demande est de décrire des suspensions de collagène formant *in vivo* des matrices de collagène stables dans le temps, notamment résistantes dans le temps aux collagénases.

Un autre but de la présente demande est de décrire des suspensions de collagène de concentration élevée, pouvant être facilement injectée de par leurs propriétés rhéologiques.

Un autre but de la présente demande est de décrire des suspensions de collagène formant après traitement des matrices denses de collagène implantables.

Un autre but de la présente demande est de décrire des suspensions de collagène non dénaturé, non réticulé, ces suspensions étant dépourvues de contaminants tels que des solvants, des émulsifiants ou des réactifs chimiques.

La demande décrit une particule solide sphérique ou sphéroïde constituée essentiellement de collagène non dénaturé et non réticulé,
le diamètre de ladite particule étant compris de 0,05 à 20 µm, en particulier de 0,25 à 10 µm, plus particulièrement de 0,4 µm à 3µm.

Par sphéroïde, on entend un solide dont la forme approche celle de la sphère.

Par diamètre, on entend le diamètre de la sphère ou le plus grand diamètre du sphéroïde.

Ce diamètre peut par exemple être mesuré par microscopie électronique ou par diffusion dynamique de la lumière.

Par « constituée essentiellement de collagène », on entend une particule comprenant plus de 90% en masse de collagène, en particulier plus de 91%, 92%, 93%, 94%, 95%, 96%, 97%, 98%, 99%, 99,5% ou 99,9%.

Par « non dénaturé », on entend un collagène dont la structure secondaire en triple hélices-α est préservée.

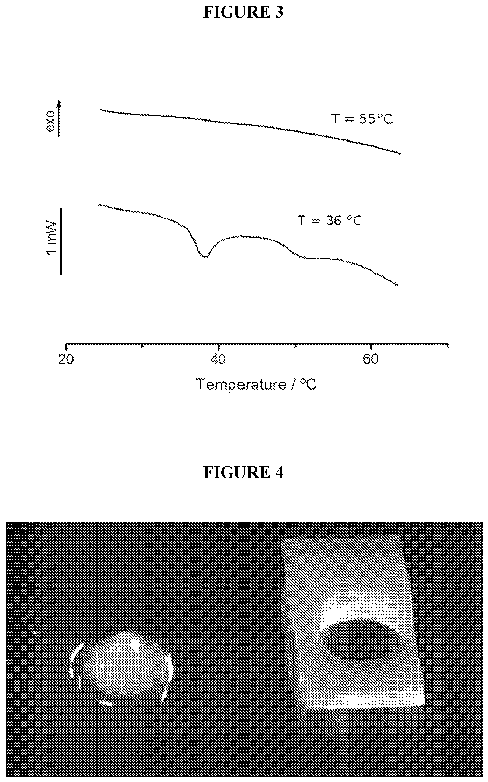

La nature non dénaturée ou dénaturée du collagène peut par exemple être observée par analyse calorimétrique. Le collagène dénaturé présente un profil calorimétrique caractéristique d'une protéine dénaturée (gélatine), sans évidence de domaines macromoléculaires organisés (figure 3).

Par « non réticulé », on entend un collagène dans lequel il n'existe pas de liaisons chimiques de réticulation, que ces liaisons soient la conséquence de modifications chimiques, comme le traitement par le glutaraldéhyde, ou de modifications physiques.

L'absence de réticulation peut par exemple être déterminée par électrophorèse.

Dans un mode particulier de la demande, celle-ci décrit une particule qui ne contient pas de contaminants tels que des solvants organiques, des émulsifiants ou des réactifs chimiques, en particulier des agents de réticulation.

Par agents de réticulation on entend de préférence le diphénylphosphorylazide (DPPA), glutaraldéhyde (GAD), un carbodiimide, comme par exemple le carbodiimide 1-éthyle -3-(3-diméthylaminopropyle) carbodiimide (EDC), le N-hydroxysuccinimide (NHS).

La source du collagène est indifférente.

En particulier, le collagène peut être obtenu d'après le protocole suivant : une solution de collagène de type I est préparée à partir de tendons de queue de rat Wistar. Après excision sous hotte à flux laminaire les tendons sont lavées dans une solution stérile de tampon phosphate saline. Les tendons sont ensuite immergés dans une solution de NaCl 4M pour éliminer les cellules intactes restantes et précipiter une partie des protéines à poids moléculaire élevé. Après lavage par la solution de tampon phosphate saline, les tendons sont solubilisés dans une solution stérile d'acide acétique 500 mM. La solution obtenue est clarifiée par centrifugation à 41000 g pendant 2 heures. Les protéines autres que le collagène sont précipitées de manière sélective dans une solution aqueuse de 300 mM NaCl et éliminées par centrifugation à 41000 g pendant 3 heures. Le collagène est récupéré à partir du surnageant par précipitation dans une solution de NaCl à 600 mM suivie d'une centrifugation à 3000 g pendant 45 minutes. Les culots obtenus sont solubilisés dans une solution aqueuse d'acide acétique 500 mM puis dyalisés dans le même solvant pour éliminer les ions de NaCl. La solution est maintenue à 4°C et centrifugée à 41000 g pendant 4 heures avant utilisation.

Le protocole qui précède peut également être appliqué aux autres de types de collagène.

Selon un mode de réalisation avantageux, la demande décrit une particule telle que définie précédemment, dans laquelle le collagène a une masse moléculaire comprise de 200 à 450 KDa.

Selon un mode de réalisation avantageux, la demande décrit une particule telle que définie précédemment, dans laquelle le collagène est choisi parmi les collagènes de type I, II, III, V, XI, XXIV, XXVII et leurs mélanges.

Selon un mode de réalisation avantageux, la demande décrit une particule telle que définie précédemment, dans laquelle le collagène est en partie au moins présent sous forme de fibrilles, préférentiellement sous forme de fibrilles striées de collagène constituées par des triples hélices de collagène et dans lesquels la périodicité est de 67 nm, lesdites fibrilles formant des domaines où les fibrilles sont alignées selon une direction préférentielle sur une grande distance, par exemple une distance supérieure à 200 nm, 500 nm, 1µm ou 5 µm, et/ou des domaines isotropes où elles ne sont pas ordonnées.

Par fibrilles, on entend un agencement ordonné de molécules de collagène donnant lieu à la formation de structures anisotropes.

Par fibrilles striées, on entend des fibrilles présentant un motif répétitif de striations lors de son observation par microscopie à transmission électronique, après ajout d'un agent de contraste tel que l'acétate d'uranyle.

Selon un mode de réalisation avantageux, la demande décritune particule telle que définie précédemment, comprenant moins de 10% en masse de solvant, notamment d'acide acétique, en particulier moins de 9%, 8%, 7%, 6%, 5%, 4%, 3%, 2%, 1%, 0,5% ou 0,1%.

La densité des particules est caractérisée par l'assemblage des molécules de collagène non réticulées dont la distance intermoléculaire est inférieure à 10 nm, de préférence inférieure à 5 nm, et en particulier inférieure à 2 nm.

Selon un mode de réalisation avantageux, la demande décrit une particule telle que définie précédemment, laquelle est biréfringente.

Un signal de biréfringence peut par exemple être observé par microscopie optique à lumière polarisée.

La demande décrit également une composition pulvérulente comprenant ou constituée des particules telles que définies précédemment.

Par composition pulvérulente, on entend la poudre formée par les particules de la demande, telles que définies précédemment.

Selon un mode de réalisation avantageux, la demande décrit une composition pulvérulente telle que définie précédemment, laquelle est dépourvue d'émulsifiant, notamment d'agents tensioactifs ou d'huiles.

Dans un mode particulier de la demande, la demande décrit une composition pulvérulente telle que définie précédemment, qui ne contient pas de contaminants tels que des solvants organiques, des émulsifiants ou des réactifs chimiques, en particulier des agents de réticulation.

La demande décrit également un procédé de préparation d'une composition pulvérulente telle que définie précédemment, comprenant une étape d'atomisation d'une solution acide de collagène non dénaturé et non réticulé à une température inférieure à environ 40°C, en particulier inférieure à environ 39°C, 38°C ou 37°C, pour obtenir la composition pulvérulente.

Lors de cette étape d'atomisation, un aérosol est formé par pulvérisation de la solution de collagène, lequel est séché pour former la composition pulvérulente.

Selon un mode de réalisation avantageux, la demande décrit un procédé tel que défini précédemment, dans lequel la concentration de la solution en collagène est comprise de 0,1 à 10 mg/ml.

Selon un mode de réalisation avantageux, la demande décrit un procédé tel que défini précédemment, dans lequel l'acide est l'acide acétique,
ladite solution aqueuse étant en particulier constituée d'eau, d'acide et de collagène non dénaturé et non réticulé,
la solution acide de collagène ayant notamment une concentration en acide acétique comprise de 0,01 à 1000 mM.

Les compositions pulvérulentes selon la demande peuvent être alternativement mises en suspension dans une solution aqueuse, une solution acide de collagène, ou une suspension de vésicules, lesquelles seront définies plus loin.

La demande décrit également une suspension comprenant ou constituée d'une composition pulvérulente telle que définie précédemment, dans une solution aqueuse.

De façon surprenante, les suspensions selon la demande peuvent être facilement injectées dans un tissu, à travers une aiguille de 27, 30 ou 32 gauge, même à haute concentration en collagène, c'est-à-dire à une concentration en collagène supérieure à 10, 20, 30, 40, 50, 60, 70, 80, 90 ou 100 mg par ml de suspension.

La solution aqueuse est notamment un tampon phosphate salin.

Selon un mode de réalisation avantageux, la demande décrit une suspension telle que définie précédemment, dans laquelle ladite solution aqueuse est une solution de collagène acido-soluble.

Par solution de collagène acido-soluble, on entend toute solution de collagène dont le pH est inférieur à 7, notamment les solutions aqueuses comprenant du collagène et de l'acide acétique.

Ainsi, dans ce cas, une composition pulvérulente de collagène selon la présente demande est mise en suspension dans une solution contenant elle-même du collagène.

Ladite suspension de collagène a une concentration en collagène plus élevée que la solution de collagène dans laquelle les particules de collagène sont ajoutées.

Selon un mode de réalisation avantageux, la demande décrit une suspension telle que définie précédemment, contenant en outre des vésicules comprenant du collagène non dénaturé et non réticulé, de l'ATP et une phase aqueuse,
la phase aqueuse des vésicules et la solution aqueuse de la suspension étant notamment de même nature, en particulier identiques.

Ladite suspension de collagène a une concentration en collagène plus élevée que la suspension de vésicules, telle que définie plus loin, dans laquelle les particules de collagène sont ajoutées.

Selon un mode de réalisation avantageux, la demande décrit une suspension telle que définie précédemment, dans laquelle la concentration en collagène est comprise de 0,1 à 200 mg par ml de suspension, en particulier de 1 à 200 mg par ml de suspension, plus particulièrement de 10 à 200 mg par ml de suspension, encore plus particulièrement de 10 à 75 mg par ml de suspension.

Par concentration en collagène, on entend la concentration totale en collagène de la suspension, c'est-à-dire des particules, et le cas échéant de la solution aqueuse ou des vésicules.

Selon un mode de réalisation avantageux, la demande décrit une suspension telle que définie précédemment, pouvant être injectée dans un tissu à travers une aiguille de 27, 30 ou 32 gauge.

La demande décrit également une composition cosmétique contenant une suspension telle que définie précédemment, ou une composition pulvérulente telle que définie précédemment.

Selon un mode de réalisation avantageux, la demande décrit une composition cosmétique telle que définie précédemment, comprenant en outre un ou plusieurs additifs choisis parmi les anesthésiants, l'acide hyaluronique, les élastines, les vitamines et du plasma riche en plaquettes, des ions, des précurseurs d'une phase minérale, des protéoglycanes, des glycosaminoglycane, des collagènes de type non-fibrillaires.

Selon un mode de réalisation avantageux, la demande décrit une composition cosmétique dans laquelle ladite suspension est une suspension comprenant ou constituée d'une composition pulvérulente telle que définie précédemment, dans une solution aqueuse de collagène acido-soluble.

La demande décrit également une composition pharmaceutique contenant à titre de substance active une suspension telle que définie précédemment, ou une composition pulvérulente telle que définie précédemment.

Selon un mode de réalisation avantageux, la demande décrit une composition pharmaceutique dans laquelle ladite suspension est une suspension comprenant ou constituée d'une composition pulvérulente telle que définie précédemment, dans une solution aqueuse de collagène acido-soluble.

Les compositions cosmétiques et pharmaceutiques de la présente demande sont en particulier non ou faiblement immunogènes.

La demande décrit également un procédé de préparation d'un matrice fibrillaire dense de collagène sous forme de gel comprenant une étape dans laquelle une suspension telle que définie précédemment est soumise à des conditions permettant d'augmenter le pH de ladite suspension à une valeur supérieure à 7.

Par matrice fibrillaire, on entend un gel physique composé par des fibrilles de collagène striées.

Par matrice fibrillaire dense, on entend notamment une matrice fibrillaire biréfringente.

Par ailleurs, une matrice fibrillaire dense est caractérisée par l'assemblage de molécules de collagène dont la distance intermoléculaire est inférieure à 10 nm, de préférence inférieure à 5 nm, et en particulier inférieure à 2 nm, la distance intermoléculaire étant notamment comprise de 1,2 à 1,8 nm.

Un signal de biréfringence peut par exemple être observé par microscopie optique à lumière polarisée.

Selon un mode de réalisation avantageux, la demande décrit un procédé tel que défini précédemment, dans lequel la matrice fibrillaire dense de collagène sous forme de gel comporte des fibrilles striées de collagène constituées par des triples hélices de collagène et dans lesquels la périodicité est de 67 nm, lesdites fibrilles formant des domaines où les fibrilles sont alignées selon une direction préférentielle sur une grande distance et/ou des domaines isotropes où elles ne sont pas ordonnées,
ledit collagène non dénaturé et non réticulé étant en particulier du collagène de type I.

De façon surprenante, des matrices fibrillaires comportant des fibrilles dans lesquelles la périodicité est de 67 nm sont obtenues, même à partir de suspensions peu concentrées en collagène, comme par exemple des suspensions à 7 mg de collagène par ml de suspension.

La demande décrit également un procédé de préparation d'un matrice fibrillaire dense de collagène sous forme de gel comprenant une étape dans laquelle une suspension telle que définie précédemment est soumise à des conditions permettant d'augmenter la force ionique de la suspension jusqu'à une valeur à laquelle la matrice fibrillaire dense de collagène sous forme de gel est formée, par exemple par mise en contact avec un milieu physiologique, notamment le liquide extracellulaire.

La demande décrit également un procédé de préparation d'un matrice fibrillaire dense de collagène sous forme de gel comprenant une étape dans laquelle une suspension telle que définie précédemment est soumise à des conditions permettant d'augmenter le pH de ladite suspension à une valeur supérieure à 7 et d'augmenter la force ionique de la suspension, jusqu'à des valeurs auxquelles laquelle la matrice fibrillaire dense de collagène sous forme de gel est formée, par exemple par mise en contact avec un milieu physiologique, notamment le liquide extracellulaire.

La demande décrit également une matrice fibrillaire dense de collagène sous forme de gel susceptible d'être obtenue par le procédé tel que défini précédemment.

La demande décrit également un châssis physiologiquement compatible, résorbable ou non, comprenant une matrice fibrillaire dense de collagène sous forme de gel tel que défini précédemment.

Les matrices fibrillaires denses de collagène sous forme de gel et les châssis de la présente demande sont en particulier non ou faiblement immunogènes.

La demande décrit également l'utilisation d'une suspension telle que définie précédemment, pour le comblement cosmétique des rides.

La demande décrit également une méthode de traitement cosmétique des rides d'un sujet, comprenant l'administration, en particulier par injection, d'une suspension telle que définie précédemment, au dit sujet.

La demande décrit également une suspension telle que définie précédemment, ou une composition pulvérulente telle que définie précédemment, pour son utilisation dans la réparation tissulaire.

Selon un mode de réalisation avantageux, la demande décrit une suspension telle que définie précédemment, ou une composition pulvérulente telle que définie précédemment, pour son utilisation dans la réparation du cartilage ou d'un disque intervertébral.

Selon un mode de réalisation avantageux, la demande décrit une suspension telle que définie précédemment, ou une composition pulvérulente telle que définie précédemment, pour son utilisation dans la réparation des articulations.

Selon un mode de réalisation avantageux, la demande décrit une suspension telle que définie précédemment, ou une composition pulvérulente telle que définie précédemment, pour son utilisation en tant que matériau de comblement, notamment en chirurgie esthétique, chirurgie uro-gynécologique, laryngoplastie ou à la suite de chirurgie percutanée.

La demande décrit également un dispositif médical implantable comprenant ou constituée d'une matrice fibrillaire dense de collagène sous forme de gel tel que défini précédemment, ou d'un châssis tel que défini précédemment, ledit dispositif étant en particulier une prothèse de disque intervertébral.

La demande décrit également une vésicule comprenant ou constitué de collagène non dénaturé et non réticulé, d'ATP et d'une phase aqueuse,
le diamètre de ladite vésicule étant compris de 0,1µm à 10µm, en particulier de 0,2µm à 5µm, plus particulièrement de 0,5 µm à 2µm, encore plus particulièrement d'environ 1µm.

Par vésicule, on entend une gouttelette flexible.

Par ATP, on entend l'adénosine-5'-triphosphate sous forme neutre ou sous l'une quelconque de ses formes chargées.

Par diamètre, on entend le diamètre de la sphère ou le plus grand diamètre du sphéroïde.

Ce diamètre peut par exemple être mesuré par microscopie ou par diffusion dynamique de la lumière.

Par « non dénaturé », on entend un collagène dont la structure secondaire en triple hélices-α est préservée.

La nature non dénaturée ou dénaturée du collagène peut par exemple être observée par analyse calorimétrique. Le collagène dénaturé présente un profil calorimétrique caractéristique d'une protéine dénaturée (gélatine), sans évidence de domaines macromoléculaires organisés (figure 3).

Par « non réticulé », on entend un collagène dans lequel il n'existe pas de liaisons chimiques de réticulation, que ces liaisons soient la conséquence de modifications chimiques, comme le traitement par le glutaraldéhyde, ou de modifications physiques.

L'absence de réticulation peut par exemple être déterminée par électrophorèse.

Dans un mode particulier de la demande, la demande décrit une particule qui ne contient pas de contaminants tels que des solvants organiques, des émulsifiants ou des réactifs chimiques, en particulier des agents de réticulation.

Par agents de réticulation on entend de préférence le diphénylphosphorylazide (DPPA), glutaraldéhyde (GAD), un carbodiimide, comme par exemple le carbodiimide 1-éthyle -3-(3-diméthylaminopropyle) carbodiimide (EDC), le N-hydroxysuccinimide (NHS).

La source du collagène est indifférente.

En particulier, le collagène peut être obtenu d'après le protocole suivant : une solution de collagène de type I est préparée à partir de tendons de queue de rat Wistar. Après excision sous hotte à flux laminaire les tendons sont lavées dans une solution stérile de tampon phosphate saline. Les tendons sont ensuite immergés dans une solution de NaCl 4M pour éliminer les cellules intactes restantes et précipiter une partie des protéines à poids moléculaire élevé. Après lavage par la solution de tampon phosphate saline, les tendons sont solubilisés dans une solution stérile d'acide acétique 500 mM. La solution obtenue est clarifiée par centrifugation à 41000 g pendant 2 heures. Les protéines autres que le collagène sont précipitées de manière sélective dans une solution aqueuse de 300 mM NaCl et éliminées par centrifugation à 41000 g pendant 3 heures. Le collagène est récupéré à partir du surnageant par précipitation dans une solution de NaCl à 600 mM suivie d'une centrifugation à 3000 g pendant 45 minutes. Les culots obtenus sont solubilisés dans une solution aqueuse d'acide acétique 500 mM puis dyalisés dans le même solvant pour éliminer les ions de NaCl. La solution est maintenue à 4°C et centrifugée à 41000 g pendant 4 heures avant utilisation.

Le protocole qui précède peut également être appliqué aux autres de types de collagène.

Selon un mode de réalisation avantageux, la demande décrit une vésicule telle que définie précédemment, dans laquelle le collagène a une masse moléculaire comprise de 200 à 450 KDa.

Selon un mode de réalisation avantageux, la demande décrit une vésicule telle que définie précédemment, dans laquelle le collagène est choisi parmi les collagènes de type I, II, III, V, XI, XXIV, XXVII et leurs mélanges.

Selon un mode de réalisation avantageux, la demande décrit une vésicule telle que définie précédemment, dans laquelle la phase aqueuse est acide, ladite phase aqueuse étant plus particulièrement constituée d'eau et d'acide,
ladite phase aqueuse comprenant en particulier de l'acide acétique, notamment à une concentration comprise de 0,00001 M à 1 M,
le pH de ladite phase aqueuse étant compris de 2,0 à 5,5, de préférence de 2,0 à 4,0.

Par phase aqueuse acide, on entend une phase aqueuse dont le pH est inférieur à 7.

Selon un mode de réalisation avantageux, la demande décrit une vésicule telle que définie précédemment, dans laquelle le collagène est sous forme segmentaire à période longue (SLS).

Par « forme segmentaire à période longue (SLS : Segment Long Spacing) », on entend un motif d'empilement du collagène, dans lequel des agrégats latéraux sont produits. Chaque agrégat a une longueur d'environ 300 nm de long, et les molécules de collagène sont toutes en registre.

Selon un mode de réalisation avantageux, la demande décrit une vésicule telle que définie précédemment, laquelle est biréfringente.

Un signal de biréfringence peut par exemple être observé par microscopie optique à lumière polarisée.

La demande décrit également une suspension comprenant ou constituée des vésicules telles que définies précédemment, dans une solution aqueuse.

Ainsi, les vésicules en suspension sont des coacervats en suspension dans ladite solution aqueuse. Ces coacervats sont des gouttelettes flexibles, dont la concentration en collagène est au moins deux fois supérieure à celle de ladite solution aqueuse.

Selon un mode de réalisation avantageux, la demande décrit une suspension telle que définie précédemment, dans laquelle ladite solution aqueuse est acide,
ladite solution aqueuse étant en particulier constituée d'eau et d'acide,
ladite solution aqueuse comprenant en particulier de l'acide acétique, notamment à une concentration comprise de 0,00001 M à 1 M,
le pH de ladite phase aqueuse étant compris de 2,0 à 5,5, de préférence de 2,0 à 4,0.

Par solution acide, on entend une solution dont le pH est inférieur à 7.

Selon un mode de réalisation avantageux, la demande décrit une suspension telle que définie précédemment, dans laquelle la phase aqueuse des vésicules et la solution aqueuse de la suspension sont de même nature, en particulier identiques.

Selon un mode de réalisation avantageux, la demande décrit une suspension telle que définie précédemment, laquelle est dépourvue d'émulsifiant, notamment d'agents tensioactifs ou d'huiles.

Selon un mode de réalisation avantageux, la demande décrit une suspension telle que définie précédemment, comprenant en outre des particules solides sphériques ou sphéroïdes constituées essentiellement de collagène non dénaturé et non réticulé,
le diamètre desdites particules étant compris de 0,05 à 20 µm, en particulier de 0,25 à 10 µm, plus particulièrement de 0,4 µm à 3µm.

Les particules solides sphériques ou sphéroïdes sont telles que définies précédemment.

Ladite suspension de collagène a une concentration en collagène plus élevée que la suspension dépourvue des particules solides sphériques ou sphéroïdes.

Selon un mode de réalisation avantageux, la demande décrit une suspension telle que définie précédemment, dans laquelle la concentration en collagène est comprise de 0,1 à 200 mg par ml de suspension, en particulier de 1 à 200 mg par ml de suspension, plus particulièrement de 10 à 200 mg par ml de suspension, encore plus particulièrement de 40 à 200 mg par ml de suspension.

Par concentration en collagène, on entend la concentration totale en collagène de la suspension, c'est-à-dire des vésicules, de la solution aqueuse et le cas échéant des particules solides sphériques ou sphéroïdes.

Selon un mode de réalisation avantageux, la demande décrit une suspension telle que définie précédemment, dans laquelle la viscosité est comprise de 1,7 à 253 mPa.s pour des concentrations en collagène de 1,45 à 28 mg par ml de suspension, respectivement.

La viscosité peut être déterminée à 50 Hz sur un rhéomètre Anton Parr MCR 302 en mode de viscosimètre rotationnel sur une géométrie cône-plan, à 25°C et sous pression atmosphérique. Le taux de cisaillement utilisé pour ces déterminations est de 50 s⁻¹.

De façon surprenante, les suspensions selon la demande peuvent être facilement injectées dans un tissu, à travers une aiguille de 27, 30 ou 32 gauge, même à haute concentration en collagène, c'est-à-dire à une concentration en collagène supérieure à 10, 20, 30, 40, 50, 60, 70, 80, 90 ou 100 mg par ml de suspension.

Selon un mode de réalisation avantageux, la demande décrit une suspension telle que définie précédemment, dans laquelle le rapport collagène / ATP est compris lors de la mise en contact de 0,1 à 10.

Selon un mode de réalisation avantageux, la demande décrit une suspension telle que définie précédemment, pouvant être injectée dans un tissu à travers une aiguille de 27, 30 ou 32 gauge.

La demande décrit également un procédé de préparation d'une suspension telle que définie précédemment, comprenant une étape de mise en contact, sous agitation, d'une solution aqueuse de collagène non dénaturé et non réticulé, avec de l'ATP, pour obtenir ladite suspension.

L'ATP peut être apporté sous forme libre ou la forme d'un sel correspondant, par exemple le sel di sodique d'ATP.

Selon un mode de réalisation avantageux, la demande décrit un procédé tel que défini précédemment, dans lequel le rapport massique collagène / ATP est compris lors de la mise en contact de 0,1 à 10.

Selon un mode de réalisation avantageux, la demande décrit un procédé tel que défini précédemment, dans lequel l'agitation se fait de 200 à 2400 tours par minute, en particulier pendant 5 à 60 secondes.

Selon un mode de réalisation avantageux, la demande décrit un procédé tel que défini précédemment, comprenant après l'étape de mise en contact, une étape de concentration de la suspension, en particulier par centrifugation de la suspension pour obtenir un culot et un surnageant, puis par élimination de tout ou partie du surnageant obtenu à l'issue de la centrifugation de la suspension, pour obtenir une suspension concentrée.

Selon un mode de réalisation avantageux, la demande décrit un procédé tel que défini précédemment, comprenant :
- une étape de mise en contact, sous agitation, d'une solution aqueuse de collagène non dénaturé et non réticulé avec de l'ATP, pour obtenir une suspension ;
- une étape de concentration de la suspension, en particulier par centrifugation de la suspension pour obtenir un culot et un surnageant, puis par élimination de tout ou partie du surnageant obtenu à l'issue de la centrifugation de la suspension, pour obtenir une suspension concentrée.

La demande décrit également une composition cosmétique contenant une suspension de vésicules comprenant du collagène non dénaturé et non réticulé, de l'ATP et une phase aqueuse, dans une solution aqueuse,
le diamètre des vésicules étant en particulier compris de 0,1µm à 10µm.

Selon un mode de réalisation avantageux, la demande décrit une composition cosmétique telle que définie précédemment, comprenant en outre un ou plusieurs additifs choisis parmi les anesthésiants, l'acide hyaluronique, les élastines, les vitamines et du plasma riche en plaquettes, des ions, des précurseurs d'une phase minérale, des protéoglycanes, des glycosaminoglycane, des collagènes de type non-fibrillaires.

Selon un mode de réalisation avantageux, la demande décrit une composition cosmétique dans laquelle ladite suspension comprend en outre des particules solides sphériques ou sphéroïdes constituées essentiellement de collagène non dénaturé et non réticulé,
le diamètre desdites particules étant compris de 0,05 à 20 µm, en particulier de 0,25 à 10 µm, plus particulièrement de 0,4 µm à 3µm.

La demande décrit également une composition pharmaceutique contenant à titre de substance active une suspension de vésicules comprenant du collagène non dénaturé et non réticulé, de l'ATP et une phase aqueuse, dans une solution aqueuse,
le diamètre des vésicules étant en particulier compris de 0,1µm à 10µm.

Selon un mode de réalisation avantageux, la demande décrit une composition pharmaceutique dans laquelle ladite suspension comprend en outre des particules solides sphériques ou sphéroïdes constituées essentiellement de collagène non dénaturé et non réticulé, le diamètre desdites particules étant compris de 0,05 à 20 µm, en particulier de 0,25 à 10 µm, plus particulièrement de 0,4 µm à 3µm.

Les compositions cosmétiques et pharmaceutiques de la présente demande sont en particulier non ou faiblement immunogènes.

La demande décrit également un procédé de préparation d'une matrice fibrillaire dense de collagène sous forme de gel comprenant une étape dans laquelle une suspension de vésicules comprenant du collagène non dénaturé et non réticulé, de l'ATP et une phase aqueuse, dans une solution aqueuse, est soumise à des conditions permettant d'augmenter le pH de ladite suspension à une valeur supérieure à 7,
le diamètre des vésicules étant en particulier compris de 0,1µm à 10µm.

Par fibrilles, on entend un agencement ordonné de molécules de collagène donnant lieu à la formation de structures anisotropes.

Par fibrilles striées, on entend des fibrilles présentant un motif répétitif de striations lors de son observation par microscopie à transmission électronique, après ajout d'un agent de contraste tel que l'acétate d'uranyle.

Par matrice fibrillaire dense, on entend notamment une matrice fibrillaire biréfringente.

Un signal de biréfringence peut par exemple être observé par microscopie optique à lumière polarisée.

Par ailleurs, une matrice fibrillaire dense est caractérisée par l'assemblage de molécules de collagène dont la distance intermoléculaire est inférieure à 10 nm, de préférence inférieure à 5 nm, et en particulier inférieure à 2 nm, la distance intermoléculaire étant notamment comprise de 1,2 à 1,8 nm.

Selon un mode de réalisation avantageux, la demande décrit un procédé tel que défini précédemment, dans lequel la matrice fibrillaire dense de collagène sous forme de gel comporte des fibrilles striées de collagène constituées par des triples hélices de collagène et dans lesquels la périodicité est de 67 nm, lesdites fibrilles formant des domaines où les fibrilles sont alignées selon une direction préférentielle sur une grande distance, par exemple une distance supérieure à 200 nm, 500 nm, 1µm ou 5 µm, et/ou des domaines isotropes où elles ne sont pas ordonnées, ledit procédé comprenant une étape dans laquelle une suspension de vésicules comprenant du collagène non dénaturé et non réticulé, en particulier du collagène fibrillaire, de préférence du collagène de type I, de l'ATP et une phase aqueuse, dans une solution aqueuse, est soumise à des conditions permettant d'augmenter le pH de ladite suspension à une valeur supérieure à 7.

De façon tout à fait surprenante, des matrices fibrillaires comportant des fibrilles dans lesquelles la périodicité est de 67 nm ont été obtenues à partir de vésicules de collagène comportant du collagène sous forme SLS.

La demande décrit également un procédé de préparation d'une matrice fibrillaire dense de collagène sous forme de gel comprenant une étape dans laquelle une suspension de vésicules comprenant du collagène non dénaturé et non réticulé, de l'ATP et une phase aqueuse, dans une solution aqueuse, est soumise à des conditions permettant d'augmenter la force ionique de la suspension jusqu'à une valeur à laquelle la matrice fibrillaire dense de collagène sous forme de gel est formée, par exemple par mise en contact avec un milieu physiologique,
le diamètre des vésicules étant en particulier compris de 0,1µm à 10µm.

La demande décrit également un procédé de préparation d'une matrice fibrillaire dense de collagène sous forme de gel comprenant une étape dans laquelle une suspension de vésicules comprenant du collagène non dénaturé et non réticulé, de l'ATP et une phase aqueuse, dans une solution aqueuse, est soumise à des conditions permettant d'augmenter le pH de ladite suspension à une valeur supérieure à 7 et d'augmenter la force ionique de la suspension jusqu'à des valeurs auxquelles la matrice fibrillaire dense de collagène sous forme de gel est formée, par exemple par mise en contact avec un milieu physiologique,
le diamètre des vésicules étant en particulier compris de 0,1µm à 10µm.

La demande décrit également une matrice fibrillaire dense de collagène sous forme de gel susceptible d'être obtenue par le procédé tel que défini précédemment.

La demande décrit également un châssis physiologiquement compatible, résorbable ou non, comprenant une matrice fibrillaire dense de collagène sous forme de gel tel que défini précédemment.

Les matrices fibrillaires denses de collagène sous forme de gel et les châssis de la présente demande sont en particulier non ou faiblement immunogènes.

La demande décrit également l'utilisation d'une suspension de vésicules comprenant ou constituées de collagène non dénaturé et non réticulé, d'ATP et d'une phase aqueuse, dans une solution aqueuse, pour le comblement cosmétique des rides,
le diamètre des vésicules étant en particulier compris de 0,1µm à 10µm.

La demande décrit également une méthode de traitement cosmétique des rides d'un sujet, comprenant l'administration, en particulier par injection, d'une suspension de vésicules comprenant ou constituées de collagène non dénaturé et non réticulé, d'ATP et d'une phase aqueuse, dans une solution aqueuse, au dit sujet,
le diamètre des vésicules étant en particulier compris de 0,1µm à 10µm.

La demande décrit également une suspension de vésicules comprenant ou constitué de collagène non dénaturé et non réticulé, d'ATP et d'une phase aqueuse, dans une solution aqueuse, pour son utilisation dans la réparation tissulaire,
le diamètre des vésicules étant en particulier compris de 0,1µm à 10µm.

Selon un mode de réalisation avantageux, la demande décrit une suspension telle que définie précédemment, pour son utilisation dans la réparation du cartilage ou d'un disque intervertébral.

Selon un mode de réalisation avantageux, la demande décrit une suspension telle que définie précédemment, pour son utilisation dans la réparation des articulations.

Selon un mode de réalisation avantageux, la demande décrit une suspension telle que définie précédemment, pour son utilisation en tant que matériau de comblement, notamment en chirurgie esthétique, chirurgie uro-gynécologique, laryngoplastie ou à la suite de chirurgie percutanée.

La demande décrit également un dispositif médical implantable comprenant ou constitué d'une matrice fibrillaire dense de collagène sous forme de gel tel que défini précédemment, ou d'un châssis tel que défini précédemment, ledit dispositif étant en particulier une prothèse de disque intervertébral.

La présente invention concerne une suspension injectable constituée d'une composition pulvérulente constituée de particules solides sphériques ou sphéroïdes constituées de plus de 90% en masse de collagène non dénaturé et non réticulé, et moins de 10% en masse d'une solution aqueuse acide, le diamètre de ladite particule étant compris de 0,05 à 20 µm, dans une solution aqueuse, et la concentration en collagène étant comprise de 1 à 200 mg par ml de suspension, pour son utilisation en tant que matériau de comblement en chirurgie esthétique, chirurgie uro-gynécologique, laryngoplastie, ou pour son utilisation dans la réparation du cartilage, la réparation des articulations ou d'un disque intervertébral.

La présente invention concerne également une suspension telle que décrite ci-dessus dans laquelle la concentration en collagène est comprise de 10 à 75 mg par ml de suspension.

La présente invention concerne également une composition cosmétique comprenant la suspension telle que décrite ci-dessus,
et un ou plusieurs additifs choisis parmi les anesthésiants, l'acide hyaluronique, les élastines, les vitamines et du plasma riche en plaquettes, des ions, des précurseurs d'une phase minérale, des protéoglycanes, des glycosaminoglycane, des collagènes de type non-fibrillaires, pour son utilisation en tant que matériau de comblement cosmétique des rides.

La présente invention concerne également une composition pharmaceutique comprenant à titre de substance active la suspension telle que décrite ci-dessus, pour son utilisation en tant que matériau de comblement en chirurgie esthétique, chirurgie uro-gynécologique, laryngoplastie ou pour son utilisation dans la réparation du cartilage, la réparation des articulations ou d'un disque intervertébral.

### DESCRIPTION DES FIGURES

La **figure 1** représente des microparticules de collagène obtenues par séchage aérosol d'une solution de collagène acido-soluble (7 mg par mL) dans l'acide acétique (0,5M) à 36 °C.
La **figure 2** représente des microparticules de collagène obtenues par séchage aérosol d'une solution de collagène acido-soluble (2,1 mg par mL) dans l'acide acétique (0,5M) à 36 °C.
La **figure 3** illustre la calorimétrie différentielle à balayage des poudres de collagène obtenues à partir du séchage par aérosol d'une solution de collagène acido-soluble (0.7 mg.mL-1) dans l'acide acétique (0,5M) à 36 et 55°C. L'analyse des échantillons a été effectuée en conditions d'humidité contrôlée pour permettre l'identification des processus de dénaturation du collagène.
La **figure 4** représente un gel de collagène obtenu par diffusion des vapeurs d'ammoniaque dans un moule de PDMS rempli avec un coacervat de collagène à une concentration de 5,6 mg.ml⁻¹ en présence d'ATP.
La **figure 5** est un cliché de microscopie électronique à transmission d'une coupe obtenue par ultramicrotomie d'un échantillon synthétisé à partir de collagène acido-soluble à 5,6 mg.mL-1 après coacervation par l'ATP et fibrillogenèse induite par des vapeurs d'ammoniaque. L'échantillon a été fixé dans une solution aqueuse de glutaraldéhyde, déshydraté, séché au point supercritique, inclut dans une matrice époxy et contrasté par de l'acétate d'uranyle.
La **figure 6** représente la viscosité des solutions de collagène acido-soluble et des coacervats de collagène selon l'exemple 8 en fonction de la concentration totale de collagène.
La **figure 7** représente la distribution de diamètre des coacervats de collagène 0,32 mg par mL selon l'exemple 9, mesuré par diffusion dynamique de la lumière.
La **figure 8** est un cliché d'une composition pulvérulente selon la demande, en microscopie optique à lumière polarisée.

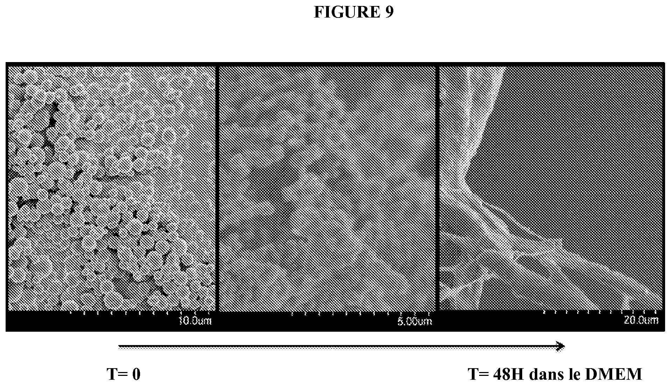

La **figure 9** présente différents états d'agrégation d'une suspension comprenant ou constituée d'une composition pulvérulente selon la demande, dans un milieu de culture Dulbecco 1X (DMEM), de t=0 à t= 48H après mise en contact de ladite suspension avec le DMEM.

### EXEMPLES

### Exemple 1 : Suspension injectable de microparticules de collagène (50 mg par mL) obtenues par voie aérosol dispersées dans une solution de collagène acido-soluble (2 mg par mL).

1.1 Obtention d'une solution de collagène acido-soluble.

Une solution de collagène de type I est préparée à partir de tendons de queue de rat Wistar. Après excision sous hotte à flux laminaire les tendons sont lavées dans une solution stérile de tampon phosphate saline. Les tendons sont ensuite immergés dans une solution de NaCl 4M pour éliminer les cellules intactes restantes et précipiter une partie des protéines à poids moléculaire élevé. Après lavage par la solution de tampon phosphate saline, les tendons sont solubilisés dans une solution stérile d'acide acétique 500 mM. La solution obtenue est clarifiée par centrifugation à 41000 g pendant 2 heures. Les protéines autres que le collagène sont précipitées de manière sélective dans une solution aqueuse de 300 mM NaCl et éliminées par centrifugation à 41000 g pendant 3 heures. Le collagène est récupéré à partir du surnageant par précipitation dans une solution de NaCl à 600 mM suivie d'une centrifugation à 3000 g pendant 45 minutes. Les culots obtenus sont solubilisés dans une solution aqueuse d'acide acétique 500 mM puis dyalisés dans le même solvant pour éliminer les ions de NaCl. La solution est maintenue à 4°C et centrifugée à 41000 g pendant 4 heures avant utilisation.

1.2 Préparation d'une poudre sèche de collagène non dénaturé.

Une solution de collagène de concentration de 7 mg par mL est diluée dans l'eau ultrapure (résistivité supérieure à 18,2 MΩ par cm) pour obtenir une solution de collagène à 1mg par mL dissoute dans une solution aqueuse d'acide acétique 71 mM. La solution ainsi obtenue est séchée dans un atomiseur (« spray drier » Buchi B290). La vitesse d'injection de la solution de collagène, limitée à 1ml par minute, est contrôlée par un pousse seringue automatique connecté à la buse de l'instrument de séchage. La température de la buse est maintenue à 36°C et la température interne du système, mesurée entre la colonne de séchage et le cyclone de collection des particules, est suivie pendant toute la durée du procès entre 26 et 31°C. Le flux d'air responsable pour le cisaillement des gouttelettes en sortie de la buse est de 1052 L par heure. Le pouvoir d'aspiration, qui contrôle le séchage des gouttelettes entre la sortie de la buse et le collecteur, est fixé à 75% de la capacité maximale du système de séchage (30 m³ par heure). Les particules ainsi formées sont collectées par un cyclone de haute performance.

1.3 Préparation d'une suspension obtenue par dispersion de la poudre de collagène dans une solution de collagène acido-soluble.

Une fraction de la poudre ainsi obtenue de masse 5,0 mg est dispersé dans 100 µL de solution de collagène acido-soluble à une concentration initiale de 2 mg par mL. La concentration finale en collagène de la suspension est de 52 mg par ml. La suspension obtenue après agitation manuelle initiale et 30 secondes d'agitation en vortex (2400 tours par minute) est aspirable par une seringue équipée d'une aiguille de 27 gauge. La suspension présente une couleur blanche et un aspect macroscopique homogène.

### Exemple 2 : Suspension injectable de microparticules de collagène (7 mg par mL) obtenues par voie aérosol dispersées dans une solution de collagène acido-soluble (7 mg par mL).

2.1 Préparation d'une poudre sèche de collagène non dénaturé.

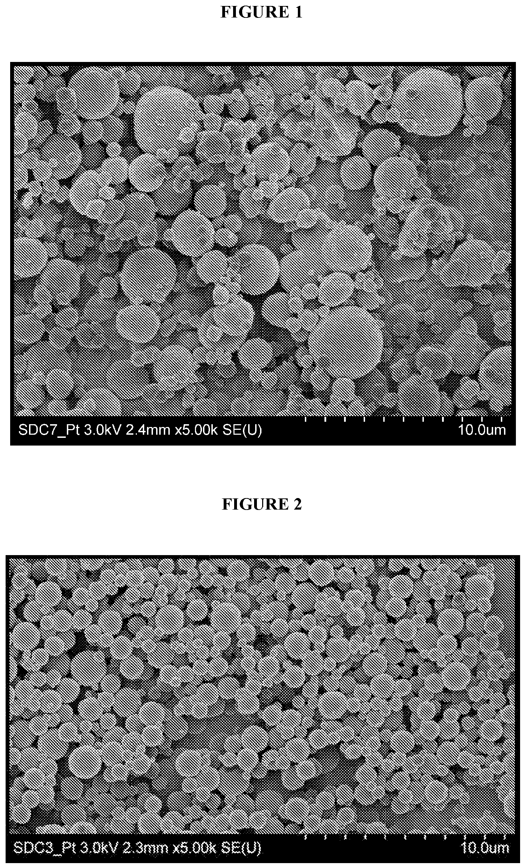

Une solution de collagène est obtenue selon l'exemple 1.1 de concentration 7 mg par mL. Cette solution est séchée dans un atomiseur (« spray drier » Buchi B290). La vitesse d'injection de la solution de collagène, limitée à 1ml par minute, est contrôlée par un pousse seringue automatique connecté à la buse de l'instrument de séchage. La température de la buse est maintenue à 36°C et la température interne du système, mesurée entre la colonne de séchage et le cyclone de collection des particules, est suivie pendant toute la durée du procès entre 26 et 31°C. Le flux d'air responsable pour le cisaillement des gouttelettes en sortie de la buse est de 1052 L par heure. Le pouvoir d'aspiration, qui contrôle le séchage des gouttelettes entre la sortie de la buse et le collecteur, est fixé à 75% de la capacité maximale du système de séchage (30 m³ par heure). Les particules ainsi formées sont collectées par un cyclone de haute performance (**figure 1**).

2.2 Préparation d'une suspension obtenue par dispersion de la poudre de collagène dans une solution de collagène acido-soluble.

Une fraction de la poudre ainsi obtenue de masse 7,0 mg est dispersé dans 1 mL de solution de collagène acido-soluble à une concentration initiale de 7 mg par mL. La concentration finale en collagène de la suspension est de 14 mg par ml. La suspension obtenue après agitation manuelle initiale et 30 secondes d'agitation en vortex (2400 tours par minute) est aspirable par une seringue équipée d'une aiguille de 27 gauge. La suspension présente une couleur blanche et un aspect macroscopique homogène.

2.3 Fibrillogenèse induite par expositions aux vapeurs d'ammoniaque.

Après injection de la suspension dans le moule de PDMS fermé, l'ensemble est exposé à des vapeurs d'ammoniaque pour favoriser la fibrillogenèse. Après 24 heures le gel ainsi formé est démoulé, sans variation de volume notable.

### Exemple 3 : Suspension injectable de microparticules de collagène (7 mg par mL) obtenues par voie aérosol dispersées dans un tampon phosphate salin.

3.1 Préparation d'une suspension obtenue par dispersion d'une poudre de collagène dans un tampon phosphate salin.

Une poudre sèche de collagène est obtenue selon l'exemple 2.2. Une fraction de la poudre ainsi obtenue de masse 7,0 mg est dispersé dans 1 mL de tampon phosphate salin. La concentration finale en collagène de la suspension est de 7 mg par ml. La suspension obtenue après agitation manuelle initiale et 30 secondes d'agitation en vortex (2400 tours par minute) est aspirable par une seringue équipée d'une aiguille de 27 gauge. La suspension présente une couleur blanche et un aspect macroscopique homogène. La suspension est ensuite injectée dans un moule cylindrique en PDMS de 70 µL.

3.2 Fibrillogenèse induite par expositions aux vapeurs d'ammoniaque.

Après injection de la suspension dans le moule de PDMS fermé, l'ensemble est exposé à des vapeurs d'ammoniaque pour favoriser la fibrillogenèse. Après 24 heures le gel ainsi formé est démoulé, sans variation de volume notable.

### Exemple 4 : Suspension injectable de microparticules de collagène (40 mg par mL) obtenues par voie aérosol dispersées dans un tampon phosphate salin.

4.1 Préparation d'une suspension obtenue par dispersion de la poudre de collagène dans tampon phosphate salin.

Une suspension de microparticules de collagène est obtenue selon exemple 3.2. Une fraction de la poudre ainsi obtenue de masse 40 mg est dispersé dans 1 mL de tampon phosphate salin. La concentration finale en collagène de la suspension est de 40 mg par ml.

4.2 Après injection de la suspension dans le moule de PDMS fermé, l'ensemble est exposé à des vapeurs d'ammoniaque pour favoriser la fibrillogenèse. Après 24 heures le gel ainsi formé est démoulé, sans variation de volume notable.

### Exemple 5 : Obtention de microparticules de collagène par voie aérosol à partir d'une solution de collagène acido-soluble à 2,1 mg par mL.

5.1 Préparation d'une poudre sèche de collagène non dénaturé.

Une solution de collagène est obtenue selon l'exemple 1.1 de concentration 7 mg par mL. Cette solution est diluée dans l'acide acétique 500 mM pour obtenir une concentration finale de 2,1 mg par mL. Ladite solution est séchée dans un atomiseur (« spray drier » Buchi B290). La vitesse d'injection de la solution de collagène, limitée à 1ml par minute, est contrôlée par un pousse seringue automatique connecté à la buse de l'instrument de séchage. La température de la buse est maintenue à 36°C et la température interne du système, mesurée entre la colonne de séchage et le cyclone de collection des particules, est suivie pendant toute la durée du procès 28 et 32°C. Le flux d'air responsable pour le cisaillement des gouttelettes en sortie de la buse est de 1052 L par heure. Le pouvoir d'aspiration, qui contrôle le séchage des gouttelettes entre la sortie de la buse et le collecteur, est fixé à 75% de la capacité maximale du système de séchage (30 m³ par heure). Les particules ainsi formées sont collectées par un cyclone de haute performance (**figure 2**).

### Exemple 6 : Analyse de microparticules de collagène non dénaturées et dénaturées obtenues par voie aérosol à partir d'une solution de collagène acido-soluble 0,7 mg par mL.

6.1 Préparation d'une poudre sèche de collagène non dénaturé selon la demande.

Une solution de collagène est obtenue selon l'exemple 1.1 de concentration 7 mg par mL. Cette solution est diluée dans l'acide acétique 500 mM pour obtenir une concentration finale de 0,7mg par mL. Ladite solution est séchée dans un atomiseur (« spray drier » Buchi B290). La vitesse d'injection de la solution de collagène, limitée à 1ml par minute, est contrôlée par un pousse seringue automatique connecté à la buse de l'instrument de séchage. La température de la buse est maintenue à 36°C et la température interne du système, mesurée entre la colonne de séchage et le cyclone de collection des particules, est suivie pendant toute la durée du procès 28 et 32°C. Le flux d'air responsable pour le cisaillement des gouttelettes en sortie de la buse est de 1052 L par heure. Le pouvoir d'aspiration, qui contrôle le séchage des gouttelettes entre la sortie de la buse et le collecteur, est fixé à 75% de la capacité maximale du système de séchage (30 m³ par heure). Les particules ainsi formées sont collectées par un cyclone de haute performance.

6.2 Préparation d'une poudre sèche de collagène dénaturé n'appartenant pas à la demande.

La solution de collagène de l'exemple 6.1 est séchée selon l'exemple 6.1 ayant comme seule différence la température de la buse, maintenue à 55 °C (**figure 3**).

### Exemple 7 : Suspension injectable de coacervats de collagène obtenue en présence de sel dissodique d'ATP

7.1 Obtention d'une suspension injectable de coacervats

A 1 mL d'une solution de collagène 7 mg par mL obtenue selon l'exemple 1.1 s'additionnent 250 µL d'une solution aqueuse de sel dissodique d'adénosine triphosphate 50 mM. Le mélange ainsi obtenu est agité sous vortex à 2400 tours par minute pendant 30 secondes. La suspension turbide ainsi obtenue est injectée dans un moule de PDMS de 70 µL.

7.2 Fibrillogenèse de la suspension de coacervats induite par expositions aux vapeurs d'ammoniaque.

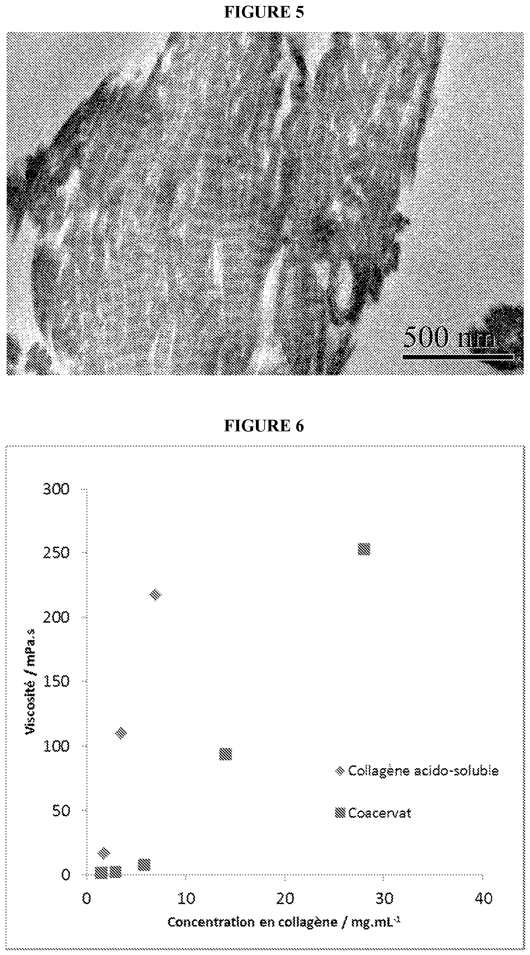

Après injection de la suspension dans le moule de PDMS fermé, l'ensemble est exposé à des vapeurs d'ammoniaque pour favoriser la fibrillogenèse. Après 24 heures le gel ainsi formé est démoulé sans de variation de volume notable (**figures 4** **et** **5**).

### Exemple 8 : Suspensions injectables de coacervats de collagène obtenues en présence de sel dissodique d'ATP

### 8.1 Obtention d'une suspension injectable de coacervats de concentration 28 mg par mL

A 1 mL d'une solution de collagène 7 mg par mL obtenue selon l'exemple 1.1 s'additionnent 200 µL d'une solution aqueuse de sel dissodique d'adénosine triphosphate 50 mM. Le mélange ainsi obtenu est agité sous vortex à 2400 tours par minute pendant 30 secondes. La suspension turbide ainsi obtenue est centrifugée pendant 2 minutes à 1700 g. Un volume de 950 µL de surneageant est enlevé et le culot ainsi obtenu agité sous un vortex de façon à obtenir un coacervat à une concentration finale de 28 mg par mL. Le coacervat ainsi obtenu est injectable dans un moule en PDMS.

### 8.2 Obtention d'une suspension injectable de coacervats de concentration 14 mg par mL

A 1 mL d'une solution de collagène 7 mg par mL obtenue selon l'exemple 1.1 s'additionnent 200 µL d'une solution aqueuse de sel dissodique d'adénosine triphosphate 50 mM. Le mélange ainsi obtenu est agité sous vortex à 2400 tours par minute pendant 30 secondes. La suspension turbide ainsi obtenue est centrifugée pendant 30 secondes à 1700 g. Un volume de 700 µL de surneageant est enlevé et le culot ainsi obtenu agité sous un vortex de façon à obtenir un coacervat à une concentration finale de 14 mg par mL. Le coacervat ainsi obtenu est injectable dans un moule en PDMS. La suspension obtenue est aussi injectable dans un gel de gélatine de type A (force Bloom 300) 10 % en masse dissout dans tampon phosphate.

### 8.3 Obtention d'une suspension injectable de coacervats de concentration 5,8 mg par mL

A 1 mL d'une solution de collagène 7 mg par mL obtenue selon l'exemple 1.1 s'additionnent 200 µL d'une solution aqueuse de sel dissodique d'adénosine triphosphate 50 mM. Le mélange ainsi obtenu est agité sous vortex à 2400 tours par minute pendant 30 secondes. La suspension turbide ainsi obtenue est de concentration finale de 5,8 mg par mL.

### 8.4 Obtention d'une suspension injectable de coacervats de concentration 2,9 mg par mL

A 500 µL d'une solution de collagène 7 mg par mL obtenue selon l'exemple 1.1 s'additionnent 500 µL d'acide acétique 500 mM et 200 µL d'une solution aqueuse de sel dissodique d'adénosine triphosphate 50 mM. Le mélange ainsi obtenu est agité sous vortex à 2400 tours par minute pendant 30 secondes. La suspension turbide ainsi obtenue est de concentration finale de 2,9 mg par mL.

### 8.5 Obtention d'une suspension injectable de coacervats de concentration 1,46 mg par mL

A 250 µL d'une solution de collagène 7 mg par mL obtenue selon l'exemple 1.1 s'additionnent 750 µL d'acide acétique 500 mM et 200 µL d'une solution aqueuse de sel dissodique d'adénosine triphosphate 50 mM. Le mélange ainsi obtenu est agité sous vortex à 2400 tours par minute pendant 30 secondes. La suspension turbide ainsi obtenue est de concentration finale de 1,46 mg par mL.

### 8.6 Analyse de la viscosité des coacervats obtenues et de solutions de collagène acido-soluble non-coacervées

La viscosité des suspensions de coacervats obtenues selon les exemples 8.1 à 8.5 a été mesurée dans un rhéomètre Anton Parr MCR 302 en mode de viscosimètre rotationnel sur une géométrie de type cône-plan. La viscosité a été déterminée à des taux de cisaillement de 1 à 100 s⁻¹. La viscosité des suspensions de coacervat à taux de cisaillement 50 s⁻¹ a été déterminé en 1,7 à 253 mPa.s pour des concentrations en collagène de 1,45 à 28 mg par ml, respectivement. La viscosité de solutions de collagène acido-soluble à des concentrations de 1 à 7 mg par mL a aussi été déterminée. Les valeurs de viscosité obtenues selon les mêmes conditions d'analyse varient entre 16,8 mPa.s pour une concentration en collagène de 1 mg par mL à 217 mPa.s pour une concentration en collagène de 7 mg par mL. La **figure 6** regroupe les données de viscosité en fonctions de la concentration de collagène pour les solutions de collagène dissout dans l'acide acétique 500 mM et les coacervats de collagène.

### Exemple 9 : Suspension de coacervats de collagène obtenues en présence de sel dissodique d'ATP

### 9.1 Observation d'une suspension injectable de coacervats de concentration 0,83 mg par mL

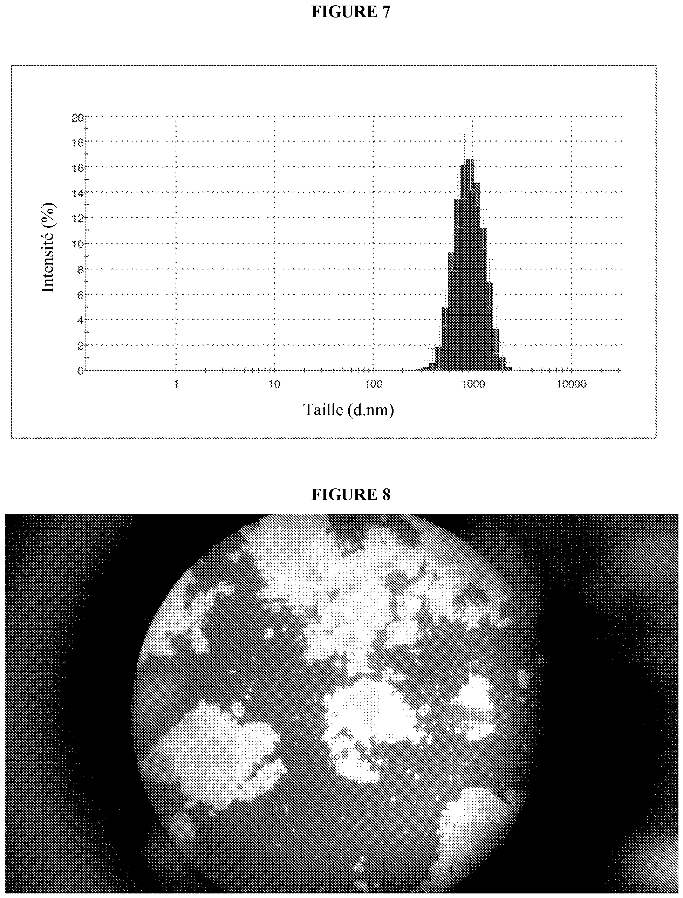

A 1mL d'une solution de collagène 0,35 mg par mL obtenue selon l'exemple 1.1 s'additionnent 100 µL d'une solution aqueuse de sel dissodique d'adénosine triphosphate 50 mM. Le mélange ainsi obtenu est agité sous vortex à 2400 tours par minute pendant 30 secondes. La suspension turbide ainsi obtenue, de concentration finale de 0,32 mg par mL est analysée par diffusion dynamique de lumière dans un appareil Zetasizer Nano S. La distribution de diamètre des particules déterminée en intensité après 5 mesures consécutives est centrée à 837 nm avec une déviation standard de 21 nm (**figure 7**).

### Exemple 10 : Etude in vivo

Un gel obtenu à partir d'une suspension selon la présente demande (par exemple selon l'exemple 2.3, 4.2 ou 7.2) est implanté *in vivo* chez le petit animal (rat wistar) en sous-cutanée. Sont évaluées :
a) l'intégration des implants (absence d'inflammation et colonisation par des cellules de l'hôte menant notamment à la présence de cellules endothéliales) ;
b) la résorption limitée par rapport à des implants de références (notamment collagène dilué et acide hyaluronique).

La non variation du volume du gel implanté, par exemple à 15, 30 ou 60 jours d'implantation, indique que le gel est stable, en particulier vis-à-vis des collagénases.

## Revendications

1. Suspension injectable constituée d'une composition pulvérulente constituée de particules solides sphériques ou sphéroïdes constituées de plus de 90% en masse de collagène non dénaturé et non réticulé, et moins de 10% en masse d'une solution aqueuse acide, le diamètre de ladite particule étant compris de 0,05 à 20 µm, dans une solution aqueuse,
et la concentration en collagène étant comprise de 1 à 200 mg par ml de suspension,
pour son utilisation en tant que matériau de comblement en chirurgie esthétique, chirurgie uro-gynécologique, laryngoplastie ou pour son utilisation dans la réparation du cartilage, la réparation des articulations ou d'un disque intervertébral.

2. Suspension selon la revendication 1 dans laquelle la concentration en collagène est comprise de 10 à 75 mg par ml de suspension.

3. Composition cosmétique comprenant la suspension selon la revendication 1,
et un ou plusieurs additifs choisis parmi les anesthésiants, l'acide hyaluronique, les élastines, les vitamines et du plasma riche en plaquettes, des ions, des précurseurs d'une phase minérale, des protéoglycanes, des glycosaminoglycane, des collagènes de type non-fibrillaires, pour son utilisation en tant que matériau de comblement en chirurgie esthétique.

4. Composition pharmaceutique comprenant à titre de substance active la suspension selon la revendication 1, pour son utilisation en tant que matériau de comblement en chirurgie uro-gynécologique, laryngoplastie ou pour son utilisation dans la réparation du cartilage, la réparation des articulations ou d'un disque intervertébral.

## Patentansprüche

1. Injizierbare Suspension, bestehend aus einer pulverförmigen Zusammensetzung, bestehend aus festen sphärischen oder sphäroiden Partikeln, die zu mehr als 90 Massen-% aus nicht denaturiertem und nicht vernetztem Kollagen und weniger als 10 Massen-% aus einer sauren wässrigen Lösung bestehen, wobei der Durchmesser des Partikels zwischen 0,05 und 20 µm in einer wässrigen Lösung beträgt,
und wobei die Kollagenkonzentration zwischen 1 und 200 mg pro ml Suspension beträgt,
für ihre Verwendung als Füllmaterial in der ästhetischen Chirurgie, der Uro-Gynäkologie, der Laryngoplastik oder für ihre Verwendung im Aufbau von Knorpeln, im Aufbau der Gelenke oder einer Bandscheibe.

2. Suspension nach Anspruch 1, bei der die Kollagenkonzentration zwischen 10 und 75 mg pro ml Suspension liegt.

3. Kosmetische Zusammensetzung, umfassend die Suspension nach Anspruch 1 und einen oder mehrere Zusatzstoffe, ausgewählt unter den Anästhetika, der Hyaluronsäure, den Elastinen, den Vitaminen und an Plättchen reichem Plasma, Ionen, Vorläufern einer mineralischen Phase, Proteoglykanen, Glykosaminoglykanen, Kollagenen nichtfibrillären Typs, für ihre Verwendung als Füllstoff in der ästhetischen Chirurgie.

4. Pharmazeutische Zusammensetzung, umfassend als Aktivstoff die Suspension nach Anspruch 1, für ihre Verwendung als Füllstoff in der Uro-Gynäkologie, Laryngoplastik oder für ihre Verwendung im Aufbau von Knorpeln, im Aufbau der Gelenke oder einer Bandscheibe.

## Claims

1. An injectable suspension consisting of a powdered composition consisting of spherical or spheroidal solid particles consisting of more than 90% by mass of non-denatured and uncrosslinked collagen, and less than 10% by mass of an acidic aqueous solution, the diameter of said particle being from 0.05 to 20 µm, in an aqueous solution,
and the collagen concentration being from 1 to 200 mg per ml of suspension,
for use as a filling material in plastic surgery, urogynecological surgery, laryngoplasty or for use in the repair of cartilage, the repair of joints or an intervertebral disc.

2. Suspension according to claim 1 wherein the collagen concentration is from 10 to 75 mg per ml of suspension.

3. Cosmetic composition comprising the suspension according to claim 1,
and one or more additives selected from anaesthetics, hyaluronic acid, elastins, vitamins and platelet rich plasma, ions, precursors of a mineral phase, proteoglycans, glycosaminoglycan, and non-fibrillar collagen types, for use as a filling material in plastic surgery.

4. Pharmaceutical composition comprising as an active substance the suspension according to claim 1, for use as a filling material in urogynecological surgery, laryngoplasty or for use in the repair of cartilage, the repair of joints or an intervertebral disc.
